# EUROPEAN PATENT SPECIFICATION

(11) **EP 3 927 703 B1**
(45) Date of publication and mention of the grant of the patent: **05.04.2023**
(21) Application number: 20707019.4
(22) Date of filing: 18.02.2020
(51) Int. Cl.: C07D 471/04, A61K 31/4375

(54) **ANTIBACTERIALS BASED ON MONOCYCLIC FRAGMENTS COUPLED TO AMINOPIPERIDINE NAPHTHYRIDINE SCAFFOLD**
ANTIBAKTERIELLE SUBSTANZEN AUF BASIS MONOCYCLISCHER FRAGMENTE, DIE MIT AMINOPIPERIDIN-NAPHTHYRIDIN-GERÜST GEKOPPELT SIND
ANTIBACTÉRIENS À BASE DE FRAGMENTS MONOCYCLIQUES COUPLÉS À UN ÉCHAFAUDAGE DE NAPHTYRIDINE AMINOPIPÉRIDINE

(30) Priority: 19.02.2019 LU 101131
(43) Date of publication of application: 29.12.2021
(73) Proprietor: Univerza V Ljubljani, 1000 Ljubljana (SI); Kemijski Institut, 1000 Ljubljana (SI)
(72) Inventor: KOLARIC, Anja, 2276 Kog (SI); MINOVSKI, Nikola, 1360 Vrhnika (SI); ANDERLUH, Marko, 1000 Ljubljana (SI); HRAST, Martina, 1000 Ljubljana (SI)
(74) Representative: Zacco GmbH
(86) International application number: PCT/EP2020/054221
(87) International publication number: WO 2020/169593

(56) References cited:
- WO-A1-02/08224
- WO-A1-02/056882
- WO-A1-2009/128019
- WO-A2-2006/010040

## Description

### FIELD OF THE INVENTION

This invention relates to novel antibacterial compounds grounded on innovative monocyclic fragments coupled to aminopiperidine naphthyridine scaffold, which can be used in treating bacterial infections. Herein disclosed novel antibacterial compounds can also be capable of treating bacterial infections caused by resistant bacterial strains, which nowadays are difficult to treat with the existing antibacterial chemotherapy.

### BACKGROUND OF THE INVENTION

The golden age of antibiotics in which more than two hundred antibacterials have been developed since penicillin has presented the mankind with the sense of security and the therapeutic area has been considered mature. Unfortunately, in the current era of enormous and uncontrolled use of antibacterials, we are witnessing a rapid grow in the antimicrobial resistance in general, making the effectiveness of these drugs highly questionable. This post a global health concern associated with alarmingly growing number of bacterial infections, which are no longer treatable with the available medicines. Bacterial infections caused by methicillin-resistant *Staphylococcus aureus* (MRSA), *Streptococcus pneumoniae, Escherichia coli, Klebsiella pneumoniae,* non-typhoidal *Salmonella* (NTS), *Shigella* species and *Neisseria gonorrhoeae,* have been exposed as an international problem to the health care by causing high proportions of resistance with increased risk for fatal clinical outcomes, which in turn requires treatments substantiated on expensive and intrusive drugs. The rapid development of drug resistance in microbes, however, has changed the situation in such a way that the research efforts directed toward the creation of a qualitatively new generation of antibacterials are stimulated both in pharma industry and academia. Put differently, there is an urgent need for development of an utterly new class of antibacterials that are previously not used in the treatment of bacterial infections.

The international publication number WO 1999/37635 discloses compounds, i.e., antibacterials grounded on aminopiperidine quinoline scaffold and connected to cyclic or acyclic moiety for the first time. Since then, a plethora of structurally similar compounds bearing antibacterial properties emerged in the patent publications, including WO 2000/21952, WO 2001/07432, WO 2001/07433, WO 2002/056882, WO 2002/08244, WO 2004/058144, WO 2006/020561, WO 2007/016610, and WO 2007/118130. While aminopiperidine linker bicyclic moiety (as disclosed in WO2006/010040, WO02/056882 and WO02/08224) prevailed as a pivotal component of the described compounds, a profusion of quinoline-like variants (e.g., napthyridines, quinoxalines, etc.) was introduced as well.

In contrast to bicyclic constructs, the present invention introduces novel compounds as antibacterials based on innovative monocyclic fragments coupled to aminopiperidine naphthyridine scaffold. By replacing bicyclic moiety of the existing antibacterials with monocyclic entities, we developed compounds with outstanding antibacterial activity and potency that outperform known antibacterial agents from the same class.

### SUMMARY OF THE INVENTION

The present invention provides novel compounds grounded on monocyclic fragments coupled to aminopiperidine naphthyridine scaffold, pharmaceutical compositions comprising said compounds, and medical uses thereof.

The present invention can be summarized as follows:
1. A compound of Formula (I): 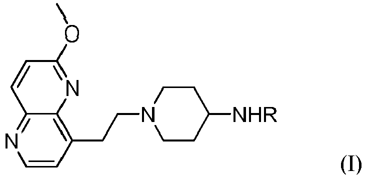 wherein R is a -CH₂-R₁ group with R₁ being a substituted or unsubstituted monocyclic aryl or a substituted or unsubstituted monocyclic aromatic heterocycle;
   or a corresponding salt, solvate, hydrate, or oxide thereof.
2. Compound according to item 1, wherein R₁ is an unsubstituted monocyclic aryl.
3. Compound according to item 1, wherein R₁ is a monocyclic aryl, substituted with one or more substituents selected from OH, halogen, CR₃R₄R₅, NR₃R₄, OCR₃R₄R₅ and COR₆; wherein R₃, R₄ and R₅ are independently selected from H, OH, halogen and (C₁₋₆)alkyl, and R₆ is selected from H, OH, halogen, (C₁₋₆)alkyl and NR₇R₈, with R₇ and R₈ being independently selected from H, halogen and (C₁₋₆)alkyl.
4. Compound according to item 1, wherein R₁ a monocyclic aryl, preferably phenyl, substituted with one or more substituents selected from OH, halogen, CH₂OH, C(O)NH₂, NH₂, N(CH₃)₂, OCH₃ and OCHR₉R₁₀ with R₉ and R₁₀ being each halogen.
5. Compound according to item 4, wherein R₉ and R₁₀ are each fluoro.
6. Compound according to any one of items 3 to 5, wherein the monocyclic aryl is substituted in para, ortho and/or meta position.
7. Compound according to any one of items 3 to 6, wherein the monocyclic aryl is mono-substituted.
8. Compound according to any one of items 3 to 6, wherein the monocyclic aryl is mono-substituted at position 4.
9. Compound according to any one of items 3 to 6, wherein the monocyclic aryl is di-substituted.
10. Compound according to any one of items 3 to 6, wherein the monocyclic aryl is 3,4-di-substituted.
11. Compound according to any one of items 1 to 10, wherein the monocyclic aryl is phenyl.
12. Compound according to item 1, wherein R₁ is an unsubstituted monocyclic aromatic heterocycle.
13. Compound according to item 1, wherein R₁ is an unsubstituted 5- or 6- membered monocyclic aromatic heterocycle.
14. Compound according to item 1, wherein R₁ is an unsubstituted 5- membered monocyclic aromatic heterocycle.
15. Compound according to item 1, wherein R₁ is an unsubstituted 6- membered monocyclic aromatic heterocycle.
16. Compound according to item 1, wherein R₁ is a substituted monocyclic aromatic heterocycle.
17. Compound according to item 1, wherein R₁ is a substituted 5- or 6- membered monocyclic aromatic heterocycle.
18. Compound according to item 1, wherein R₁ is a substituted 5- membered monocyclic aromatic heterocycle.
19. Compound according to item 1, wherein R₁ is a substituted 6- membered monocyclic aromatic heterocycle.
20. Compound according to any one of items 16 to 19, wherein the monocyclic aromatic heterocycle is substituted with one or more substituents selected from (C₂₋₆)alkenyl, (C₄₋₆)dienyl, (C₂₋₆)alkynyl, and (C₄₋₆)diynyl.
21. Compound according to any one of items 16 to 20, wherein at least one N heteroatom in the heterocycle is substituted with one or more substituents selected from (C₂₋₆)alkenyl, (C₄₋₆)dienyl, (C₂₋₆)alkynyl, and (C₄₋₆)diynyl.
22. Compound according to any one of items 16 to 20, wherein at least two N heteroatoms in the heterocycle are substituted with one or more substituents selected from (C₂₋₆)alkenyl, (C₄₋₆)dienyl, (C₂₋₆)alkynyl, and (C₄₋₆)diynyl.
23. Compound according to any one of item 16 to 20, wherein the monocyclic aromatic heterocycle is substituted with one or more substituents selected from ethenyl, propenyl, butenyl, pentenyl and hexenyl.
24. Compound according to any one of items 16 to 20, wherein at least one N heteroatom in the heterocycle is substituted with one or more substituents selected from ethenyl, propenyl, butenyl, pentenyl and hexenyl.
25. Compound according to any one of items 16 to 20, wherein at least two N heteroatoms in the heterocycle are substituted with one or more substituents selected from ethenyl, propenyl, butenyl, pentenyl and hexenyl.
26. Compound according to any one of items 16 to 20, wherein the monocyclic aromatic heterocycle is substituted with one or more substituents selected from vinyl, 2-propenyl, 3-butenyl, 4-pentenyl and 5-hexenyl.
27. Compound according to any one of items 16 to 20, wherein at least one N heteroatom in the heterocycle is substituted with one or more substituents selected from vinyl, 2-propenyl, 3-butenyl, 4-pentenyl and 5-hexenyl.
28. Compound according to any one of items 16 to 20, wherein at least two N heteroatoms in the heterocycle are substituted with one or more substituents selected from vinyl, 2-propenyl, 3-butenyl, 4-pentenyl and 5-hexenyl.
29. Compound according to any one of items 16 to 28, wherein the monocyclic aromatic heterocycle is substituted at position 1.
30. Compound according to any one of items 16 to 29, wherein the monocyclic aromatic heterocycle is substituted at position 2.
31. Compound according to any one of items 16 to 30, wherein the monocyclic aromatic heterocycle is mono-substituted.
32. Compound according to any one of items 16 to 31, wherein the monocyclic aromatic heterocycle is mono-substituted at position 1.
33. Compound according to any one of items 16 to 31, wherein the monocyclic aromatic heterocycle is mono-substituted at position 2.
34. Compound according to any one of items 16 to 31, wherein the monocyclic aromatic heterocycle is mono-substituted at position 1 with a substituent selected from ethenyl, propenyl, butenyl, pentenyl and hexenyl.
35. Compound according to any one of items 16 to 31, wherein the monocyclic aromatic heterocycle is mono-substituted at position 2 with a substituent selected from ethenyl, propenyl, butenyl, pentenyl and hexenyl.
36. Compound according to any one of items 16 to 31, wherein the monocyclic aromatic heterocycle is mono-substituted at position 1 with a substituent selected from vinyl, 2-propenyl, 3-butenyl, 4-pentenyl or 5-hexenyl.
37. Compound according to any one of items 16 to 31, wherein the monocyclic aromatic heterocycle is mono-substituted at position 2 with a substituent selected from vinyl, 2-propenyl, 3-butenyl, 4-pentenyl or 5-hexenyl.
38. Compound according to any one of items 12 to 37, wherein the monocyclic aromatic heterocycle is an azole.
39. Compound according to any one of items 12 to 38, wherein the monocyclic aromatic heterocycle has one, two or three N heteroatoms.
40. Compound according to any one of items 12 to 39, wherein the monocyclic aromatic heterocycle has two or three N heteroatoms.
41. Compound according to any one of items 12 to 40, wherein the monocyclic aromatic heterocycle has two N heteroatoms.
42. Compound according to any one of items 12 to 41, wherein the monocyclic aromatic heterocycle is pyrazole, imidazole, 1,2,3-triazole or 1,2,4-triazole.
43. Compound according to any one of items 12 to 42, wherein the monocyclic aromatic heterocycle is pyrazole.
44. Compound according to item 15, wherein the monocyclic aromatic heterocycle is 1H-pyrazol-4-yl substituted at position 1 with (C₂₋₆)alkenyl.
45. Compound according to any one of items 1 to 44, which is selected from the following list:
   *N*-benzyl-1-(2-(6-methoxy-1,5-naphthyridin-4-yl)ethyl)piperidin-4-amine;
   (4-((1-(2-(6-methoxy-1,5-naphthyridin-4-yl)ethyl)piperidin-4-ylamino)methyl)phenyl)methanol;
   4-((1-(2-(6-methoxy-1,5-naphthyridin-4-yl)ethyl)piperidin-4-ylamino)methyl)benzamide;
   *N-*(4-(dimethylamino)benzyl)-1-(2-(6-methoxy-1,5-naphthyridin-4-yl)ethyl)piperidin-4-amine;
   *N*-(4-fluorobenzyl)-1-(2-(6-methoxy-1,5-naphthyridin-4-yl)ethyl)piperidin-4-amine;
   *N-*(4-chlorobenzyl)-1-(2-(6-methoxy-1,5-naphthyridin-4-yl)ethyl)piperidin-4-amine;
   *N*-(4-bromobenzyl)-1-(2-(6-methoxy-1,5-naphthyridin-4-yl)ethyl)piperidin-4-amine;
   *N*-(4-iodobenzyl)-1-(2-(6-methoxy-1,5-naphthyridin-4-yl)ethyl)piperidin-4-amine;
   *N-*(4-(difluoromethoxy)-3-methoxybenzyl)-1-(2-(6-methoxy-1,5-naphthyridin-4-yl)ethyl)piperidin-4-amine;
   2-(difluoromethoxy)-5 -((1 -(2-(6-methoxy-1,5-naphthyridin-4-yl)ethyl)piperidin-4-ylamino)methyl)phenol; and
   *N*-((1-allyl-1*H*-pyrazol-4-yl)methyl)-1-(2-(6-methoxy-1,5-naphthyridin-4-yl)ethyl)piperidin-4-amine.
46. Use of a compound selected from *tert*-butyl 1-(2-(6-methoxy-1,5-naphthyridin-4-yl)ethyl)piperidin-4-ylcarbamate and 1-(2-(6-methoxy-1,5-naphthyridin-4-yl)ethyl)piperidin-4-amine in the preparation of a compound according to any one of items 1 to 45.
47. A pharmaceutical composition comprising a compound as defined in any of items 1 to 45 or a pharmaceutically acceptable salt thereof, and a pharmaceutically acceptable carrier, adjuvant or vehicle.
48. A compound as defined in any of items 1 to 45 for use as a medicament; preferably for use as a medicament for the treatment or prophylaxis of a bacterial infection.
49. Use of a compound according to any one of items 1 to 45 in the preparation of a medicament.
50. Use of a compound according to any one of items 1 to 45 in the preparation of a medicament for the treatment or prophylaxis of a bacterial infection.
51. Compound for use in a method for treating or preventing a bacterial infection in a patient in need thereof, comprising administering said patient a therapeutic effective amount of a compound according to any one of items 1 to 45 or a pharmaceutical composition according to item 47.
The compounds of the present invention show high enzymatic inhibitory activity, high potency against various pathogens, and are useful as agents against bacterial infections.

### BRIEF DESCRIPTION OF THE DRAWINGS AND TABLES

Scheme 1. Synthesis of intermediate **12** and compounds **C1-11.**
Table 1*. In vitro* bacterial DNA gyrase inhibitory activity and human topo IIα selectivity.
Table 2. Antibacterial activity.
Table 3. Evaluation of the compound toxicity to the cells.

### DETAILED DESCRIPTION OF THE INVENTION

The present invention provides in first aspect a compound of Formula (I): 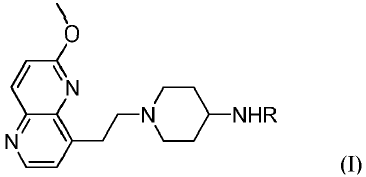 wherein R is a -CH₂-R₁ group withR₁ being a substituted or unsubstituted monocyclic aryl or a
substituted or unsubstituted monocyclic aromatic heterocycle.

According to some embodiments, the compound according to the invention of general formula I is a compound wherein R₁ is an unsubstituted monocyclic aryl.

According to some embodiments, the compound according to the invention of general formula I is a compound wherein R₁ is a monocyclic aryl, substituted with one or more substituents selected from OH, halogen, CR₃R₄R₅, NR₃R₄, OCR₃R₄R₅ and COR₆; wherein R₃, R₄ and R₅ are independently selected from H, OH, halogen and (C₁₋₆)alkyl, and R₆ is selected from H, OH, halogen, (C₁₋₆)alkyl and NR₇R₈, with R₇ and R₈ being independently selected from H, halogen and (C₁₋₆)alkyl.

According to some embodiments, the compound according to the invention of general formula I is a compound wherein R₁ is a monocyclic aryl, preferably phenyl, substituted with one or more substituents selected from OH, halogen, CH₂OH, C(O)NH₂, NH₂, N(CH₃)₂, OCH₃ and OCHR₉R₁₀ with R₉ and R₁₀ being each halogen.

According to some embodiments, the compound according to the invention of general formula I is a compound wherein R₉ and R₁₀ are each fluoro.

According to some embodiments, the compound according to the invention of general formula I is a compound wherein the monocyclic aryl is substituted in para, ortho and/or meta position.

According to some embodiments, the compound according to the invention of general formula I is a compound wherein the monocyclic aryl is mono-substituted.

According to some embodiments, the compound according to the invention of general formula I is a compound wherein the monocyclic aryl is mono-substituted at position 4.

According to some embodiments, the compound according to the invention of general formula I is a compound wherein the monocyclic aryl is di-substituted.

According to some embodiments, the compound according to the invention of general formula I is a compound wherein the monocyclic aryl is 3,4-di-substituted.

According to preferred embodiments, the compound according to the invention of general formula I is a compound wherein the monocyclic aryl is phenyl.

According to some embodiments, the compound according to the invention of general formula I is a compound wherein R₁ is an unsubstituted monocyclic aromatic heterocycle.

According to some embodiments, the compound according to the invention of general formula I is a compound wherein R₁ is an unsubstituted 5- or 6- membered monocyclic aromatic heterocycle.

According to some embodiments, the compound according to the invention of general formula I is a compound wherein R₁ is an unsubstituted 5- membered monocyclic aromatic heterocycle.

According to some embodiments, the compound according to the invention of general formula I is a compound wherein R₁ is a substituted monocyclic aromatic heterocycle.

According to some embodiments, the compound according to the invention of general formula I is a compound wherein R₁ is a substituted 5- or 6- membered monocyclic aromatic heterocycle.

According to some embodiments, the compound according to the invention of general formula I is a compound wherein R₁ is an unsubstituted 6- membered monocyclic aromatic heterocycle.

According to some embodiments, the compound according to the invention of general formula I is a compound wherein R₁ is a substituted 5- membered monocyclic aromatic heterocycle.

According to some embodiments, the compound according to the invention of general formula I is a compound wherein the monocyclic aromatic heterocycle is substituted with one or more substituents selected from (C₂₋₆)alkenyl, (C₄₋₆)dienyl, (C₂₋₆)alkynyl, and (C₄₋₆)diynyl.

According to some embodiments, the compound according to the invention of general formula I is a compound wherein the monocyclic aromatic heterocycle is substituted with one or more substituents selected from ethenyl, propenyl, butenyl, pentenyl and hexenyl.

According to some embodiments, the compound according to the invention of general formula I is a compound wherein the monocyclic aromatic heterocycle is substituted with one or more substituents selected from vinyl, 2-propenyl, 3-butenyl, 4-pentenyl or 5-hexenyl.

According to some embodiments, the compound according to the invention of general formula I is a compound wherein the monocyclic aromatic heterocycle is substituted with 2-propenyl.

According to some embodiments, the compound according to the invention of general formula I is a compound wherein at least one N heteroatom (such as at least two N heteroatoms) in the heterocycle is substituted with one or more substituents selected from (C₂₋₆)alkenyl, (C₄₋₆)dienyl, (C₂₋₆)alkynyl, and (C₄₋₆)diynyl.

According to some embodiments, the compound according to the invention of general formula I is a compound wherein at least one N heteroatom (such as one, two or three N heteroatoms) in the heterocycle is substituted with one or more substituents selected from ethenyl, propenyl, butenyl, pentenyl and hexenyl.

According to some embodiments, the compound according to the invention of general formula I is a compound wherein at least one N heteroatom (such one, two or three N heteroatoms) in the heterocycle is substituted with one or more substituents selected from vinyl, 2-propenyl, 3-butenyl, 4-pentenyl and 5-hexenyl.

According to some embodiments, the compound according to the invention of general formula I is a compound wherein at least one N heteroatom (such one, two or three N heteroatoms) in the heterocycle is substituted with 2-propenyl.

According to some embodiments, the compound according to the invention of general formula I is a compound wherein at least two N heteroatoms (such as two or three N heteroatoms) in the heterocycle are substituted with one or more substituents selected from (C₂₋₆)alkenyl, (C₄₋₆)dienyl, (C₂₋₆)alkynyl, and (C₄₋₆)diynyl.

According to some embodiments, the compound according to the invention of general formula I is a compound wherein at least two N heteroatoms (such as two or three N heteroatoms) in the heterocycle are substituted with one or more substituents selected from ethenyl, propenyl, butenyl, pentenyl and hexenyl.

According to some embodiments, the compound according to the invention of general formula I is a compound wherein at least two N heteroatoms (such as two or three N heteroatoms) in the heterocycle are substituted with one or more substituents selected from vinyl, 2-propenyl, 3-butenyl, 4-pentenyl and 5-hexenyl.

According to some embodiments, the compound according to the invention of general formula I is a compound wherein at least two N heteroatom (such as two or three N heteroatoms) in the heterocycle are substituted with 2-propenyl.

According to some embodiments, the compound according to the invention of general formula I is a compound wherein the monocyclic aromatic heterocycle is substituted at position 1.

According to some embodiments, the compound according to the invention of general formula I is a compound wherein the monocyclic aromatic heterocycle is substituted at position 2.

According to some embodiments, the compound according to the invention of general formula I is a compound wherein the monocyclic aromatic heterocycle is mono-substituted.

According to some embodiments, the compound according to the invention of general formula I is a compound wherein the monocyclic aromatic heterocycle is mono-substituted at position 1.

According to some embodiments, the compound according to the invention of general formula I is a compound wherein the monocyclic aromatic heterocycle is mono-substituted at position 2.

According to some embodiments, the compound according to the invention of general formula I is a compound wherein the monocyclic aromatic heterocycle is mono-substituted at position 1 with a substituent selected from ethenyl, propenyl, butenyl, pentenyl and hexenyl.

According to some embodiments, the compound according to the invention of general formula I is a compound wherein the monocyclic aromatic heterocycle is mono-substituted at position 1 with a substituent selected from vinyl, 2-propenyl, 3-butenyl, 4-pentenyl or 5-hexenyl.

According to some embodiments, the compound according to the invention of general formula I is a compound wherein the monocyclic aromatic heterocycle is mono-substituted at position 1 with 2-propenyl.

According to some embodiments, the compound according to the invention of general formula I is a compound wherein the monocyclic aromatic heterocycle is mono-substituted at position 2 with a substituent selected from ethenyl, propenyl, butenyl, pentenyl and hexenyl.

According to some embodiments, the compound according to the invention of general formula I is a compound wherein the monocyclic aromatic heterocycle is mono-substituted at position 2 with a substituent selected from vinyl, 2-propenyl, 3-butenyl, 4-pentenyl or 5-hexenyl.

According to some embodiments, the compound according to the invention of general formula I is a compound wherein the monocyclic aromatic heterocycle is mono-substituted at position 2 with 2-propenyl.

According to some embodiments, the compound according to the invention of general formula I is a compound wherein the monocyclic aromatic heterocycle is an azole.

According to some embodiments, the compound according to the invention of general formula I is a compound wherein the monocyclic aromatic heterocycle has one, two or three N heteroatoms.

According to some embodiments, the compound according to the invention of general formula I is a compound wherein the monocyclic aromatic heterocycle has two or three N heteroatoms.

According to some embodiments, the compound according to the invention of general formula I is a compound wherein the monocyclic aromatic heterocycle has two N heteroatoms.

According to some embodiments, the compound according to the invention of general formula I is a compound wherein the monocyclic aromatic heterocycle is pyrazole, imidazole, 1,2,3-triazole or 1,2,4-triazole.

According to preferred embodiments, the compound according to the invention of general formula I is a compound wherein the monocyclic aromatic heterocycle is pyrazole.

According to some embodiments, the compound according to the invention of general formula I is a compound wherein the monocyclic aromatic heterocycle is 1*H*-pyrazol-4-yl substituted at position 1 with (C₂₋₆)alkenyl.

According to preferred embodiments the compound according to the invention of general formula I is a compound wherein, R₁ is independently
- phenyl; or
- phenyl monosubstituted at position 4 with NH₂, CH₂OH, CONH₂, N(CH₃)₂, F, Cl, Br or I; or
- phenyl disubstituted at position 3 with OH or methoxy and at position 4 with OCHF₂; or
- 1*H*-pyrazol-4-yl substituted at position 1 with allyl.

According to a preferred embodiment, the compound according to the invention of general formula I is a compound selected from the following list:
*N*-benzyl-1-(2-(6-methoxy-1,5-naphthyridin-4-yl)ethyl)piperidin-4-amine;
(4-((1-(2-(6-methoxy-1,5-naphthyridin-4-yl)ethyl)piperidin-4-ylamino)methyl)phenyl)methanol;
4-((1-(2-(6-methoxy-1,5-naphthyridin-4-yl)ethyl)piperidin-4-ylamino)methyl)benzamide;
*N-*(4-(dimethylamino)benzyl)-1-(2-(6-methoxy-1,5-naphthyridin-4-yl)ethyl)piperidin-4-amine;
*N-*(4-fluorobenzyl)-1-(2-(6-methoxy-1,5-naphthyridin-4-yl)ethyl)piperidin-4-amine;
*N-*(4-chlorobenzyl)-1-(2-(6-methoxy-1,5-naphthyridin-4-yl)ethyl)piperidin-4-amine;
*N*-(4-bromobenzyl)-1-(2-(6-methoxy-1,5-naphthyridin-4-yl)ethyl)piperidin-4-amine;
*N*-(4-iodobenzyl)-1-(2-(6-methoxy-1,5-naphthyridin-4-yl)ethyl)piperidin-4-amine;
*N-*(4-(difluoromethoxy)-3-methoxybenzyl)-1-(2-(6-methoxy-1,5-naphthyridin-4-yl)ethyl)piperidin-4-amine;
2-(difluoromethoxy)-5 -((1 -(2-(6-methoxy-1,5-naphthyridin-4-yl)ethyl)piperidin-4-ylamino)methyl)phenol; and
*N*-((1-allyl-1*H*-pyrazol-4-yl)methyl)-1-(2-(6-methoxy-1,5-naphthyridin-4-yl)ethyl)piperidin-4-amine.

In another aspect the present invention provides the use of a compound selected from tert-butyl 1-(2-(6-methoxy-1,5-naphthyridin-4-yl)ethyl)piperidin-4-ylcarbamate and 1-(2-(6-methoxy-1,5-naphthyridin-4-yl)ethyl)piperidin-4-amine in the preparation of a compound according to the present invention as described above.

In another aspect the present invention provides a pharmaceutical composition comprising a compound according to the present invention as described above or a pharmaceutically acceptable salt thereof, and a pharmaceutically acceptable carrier, adjuvant or vehicle. The present invention thus provides pharmaceutical compositions comprising a compound of this invention, or a pharmaceutically acceptable salt thereof together with a pharmaceutically acceptable carrier, adjuvant, or vehicle, for administration to a patient.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form, either solid or liquid. Suitable dose forms for oral administration may be tablets, capsules, syrups or solutions and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

The compounds and compositions of this invention may be used with other drugs to provide a combination therapy. The other drugs may form part of the same composition, or be provided as a separate composition for administration at the same time or at different time.

Another aspect of the invention refers to a compound of the invention according as described above according to general formula I, or a pharmaceutically acceptable salt thereof, for use as a medicament for the treatment or prophylaxis of a bacterial infection.

Another aspect of the invention refers to the use of a compound of the invention or a pharmaceutically acceptable salt thereof in the manufacture of a medicament.

Another aspect of the invention refers to the use of a compound of the invention in the manufacture of a medicament for the treatment or prophylaxis of a bacterial infection.

Another aspect of this invention relates to a compound for use in a method of treating or preventing a bacterial infection, comprises administering to a patient in need of such a treatment a therapeutically effective amount of a compound as above defined or a pharmaceutical composition thereof.

The term "halogen", as used herein, includes fluoro, chloro, bromo, and iodo substituents.

The term "alkyl", as used herein, refers to any linear and branched chains with number of carbons in specified range, for instance, methyl, ethyl, *n-, iso*-propyl, *n-, iso-, sec-, t*-butyl, pentyl and hexyl.

The term "alkenyl", as used herein, refers to any linear and branched chains with number of carbons in specified range, for instance, vinyl, 2-propenyl, 3-butenyl, 4-pentenyl, 5-hexenyl.

The term "dienyl", as used herein, refers to any linear and branched chains with number of carbons in specified range, for instance, 1,3-butadienyl, 1,3-pentadienyl, 1,3-hexadienyl, 1,5-hexadienyl, 3,5-hexadienyl.

The term "alkynyl", as used herein, refers to any linear and branched chains with number of carbons in specified range, for instance, ethynyl, 2-propynyl, 3-butynyl, 3-pentynyl, 4-pentynyl, 5-hexynyl.

The term "diynyl", as used herein, refers to any linear and branched chains with number of carbons in specified range, for instance, 1,3-butadiynyl, 1,3-pentadiynyl, 1,3-hexadiynyl, 1,5-hexadiynyl, 3,5-hexadiynyl.

The term "aryl", as used herein, refers to ring systems with one aromatic ring but without heteroatoms even in only one of the rings. A preferred example of an aryl is phenyl.

The term "heterocycle", as used herein, refers to an heterocyclic ring system with one aromatic containing one or more heteroatoms selected from the group consisting of nitrogen, oxygen and sulfur in the ring. A heterocyclic group can also be substituted once or several times. Preferably, the term "heterocycle", as used herein, refers to 5- or 6- membered aromatic ring containing 1, 2 or 3 heteroatoms selected from N. More preferably, the term "heterocycle", as used herein, refers to a 5-membered aromatic ring containing from 1, 2 or 3 heteroatoms selected from N. Examples of a heterocycle include pyrazole, imidazole, 1,2,3-triazole or 1,2,4-triazole. A preferred example is pyrazole. Heterocycle can be substituted with one substituent selected from (C₂₋₆)alkenyl, (C₄₋₆)dienyl, (C₂₋₆)alkynyl, (C₄₋₆)diynyl or their combinations. The term "substituted with one or more substituents", as used herein, refers to optional substitution with 1, 2, 3, 4, or 5 substituents, where all the substituents may be the same or different, or up to 4 substituents may be the same and the rest are different.

The compounds of the present invention can be found in the form of salts, such as pharmaceutically acceptable salts, solvates, hydrates, and oxides which are also covered within this invention and possess the desired activity and potency of the parent compound.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals. Physiologically acceptable salts can also be formed with anions or acids and in the context of this invention is understood as meaning salts of at least one of the compounds used according to the invention as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be present in crystalline form or in the form of free compounds like a free base.

Any compound that is a solvate of a compound according to the invention like a compound according to general formula I defined above is understood to be also covered by the scope of the invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent). Especially preferred examples include hydrates and alcoholates, like methanolates or ethanolates.

Any compound that is a prodrug of a compound according to the disclosure like a compound according to general formula I defined above is understood to be also covered by the scope of the disclosure The term "prodrug" is used in its broadest sense and encompasses those derivatives that are converted *in vivo* to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by 13C- or 14C-enriched carbon or of a nitrogen by 15N-enriched nitrogen are within the scope of this invention.

The compounds of formula (I) as well as their salts or solvates of the compounds are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts. This applies also to its solvates or prodrugs.

This invention covers also all possible stereoisomers, geometric isomers, and tautomers of the compounds presented herein. Each compound can have one or more asymmetric centers and can therefore exist as individual diastereoisomers, or enantiomers, or as a mixture of stereoisomers. The term "geometric isomers" refers to compounds containing one or more double bonds, where *cis* or *trans* isomeric form can occur. Also included are tautomeric forms of the compounds that are able to interconvert.

### Abbreviations

- ATR: attenuated total reflectance
- DCM: dichloromethane
- DMSO: dimethyl sulfoxide
- ESI: electrospray ionization
- equiv: equivalent
- HPLC: high-performance liquid chromatography
- HRMS: high-resolution mass spectrometry
- IR: infrared spectroscopy
- MeOH: methanol
- Mp: melting point
- NMR: nuclear magnetic resonance
- Rf: retention factor
- UV: ultraviolet

### General experimental details:

All commercially available compounds were used without further purification. Anhydrous conditions were achieved using anhydrous solvents under inert atmosphere. Visualization with UV light and staining reagents on Merck silica gel (60 F254) plates (0.25 mm thick) was used for reaction progress and Rf determination. Column chromatography for compound purification was performed on silica gel 60 (particle size 0.040-0.063 mm; Merck). HPLC purity was analysed on Thermo Scientific DIONEX UltiMate 3000 instrument equipped with diode array detector using Acquity UPLC^{®} BEH C8 column (1.7 µm, 2.1 mm × 50 mm) by solvent system consisting of 0.1% trifluoroacetic acid in water (A) and acetonitrile (B), following the gradient: 90% A to 50% A in 6 min, then 50% A for 3 min, with flow rate 0.3 mL/min and injection volume 5 µL. All tested compounds showed ≥95% purity. Melting points were detected on a Reichert hot stage microscope and are uncorrected. ¹H and ¹³C NMR spectra were recorded at 400 and 100 MHz, respectively, on a Bruker AVANCE III spectrometer (Bruker Corporation, Billerica, MA, USA) in DMSO-d₆ or CDCl₃ solution using tetramethylsilane as internal standard. Mass spectra were recorded on Advion CMS spectrometer (Advion Inc., Ithaca, USA) or VG Analytical Autospec Q mass spectrometer (Fisons, VG Analytical, Manchester, UK). For obtaining IR spectra, Thermo Nicolet Nexus 470 ESP FT-IR spectrometer (Thermo Fisher Scientific, Waltham, MA, USA) was used.

### General synthetic scheme:

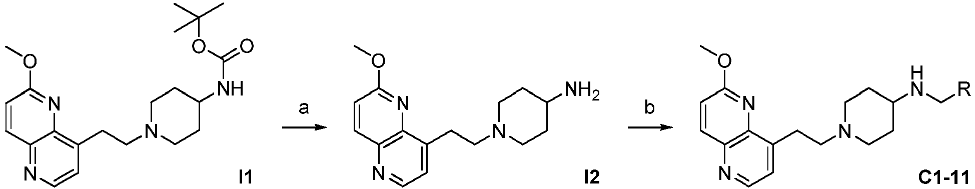

### Reagents:

a) (i) 1M HCl/CH₃COOH, 30 min, rt, (ii) 1M NaOH
b) (i) various aldehydes, CH₃COOH, MeOH, 2h, rt, (ii) NaCNBH₃, 0 °C, (iii) overnight, rt

Scheme 1. Synthesis of intermediate **12** and compounds **C1-11.**

### General procedure for reductive amination:

To a solution of intermediate **I2** (1 equiv) in dry methanol various aldehydes (1.05-1.3 equiv) were added, together with catalytic amount of acetic acid. The reaction mixture was stirred for 2h at room temperature under inert atmosphere. Next, the reaction mixture was cooled to 0 °C and NaCNBH₃ (3.5-4 equiv) dissolved in small amount of dry methanol was added drop-wise. After stirring overnight at room temperature under inert atmosphere, the solvent was evaporated in vacuum and the residue diluted in ethyl acetate (20 mL). Organic phase was washed with saturated Na₂CO₃ aqueous solution (3 × 10 mL), dried over Na₂SO₄, filtered and the solvent evaporated under reduced pressure. The residue was purified by flash column chromatography to afford appropriate product.

### Example 1 - Intermediate I2

### 1-(2-(6-methoxy-1,5-naphthyridin-4-yl)ethyl)piperidin-4-amine

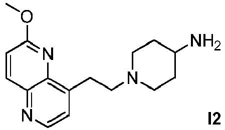

*Tert-butyl* 1-(2-(6-methoxy-1,5-naphthyridin-4-yl)ethyl)piperidin-4-ylcarbamate **(I1)** was prepared by method described in WO 2001/07432 A2.

**I1** (0.63g, 1.63 mmol, 1 equiv) was dissolved in 1 M HCl in acetic acid (12.5 mL) and stirred for 30 min at room temperature. Solvent was evaporated, the residue dissolved in saturated NaHCO₃ (30 mL) and pH adjusted to 12 with 1M NaOH. Water layer was washed with DCM (3 × 30 mL), combined organic layers dried over Na₂SO₄ and concentrated in vacuum to afford intermediate **I2** as dark-brown viscous liquid (0.330 g, 70.7%).
**Rf:** 0.10 (DCM/MeOH 4:1 + 1% Et₃N);
**¹H NMR (400 MHz, CDCl₃):** δ = 1.41-1.51 (m, 2H), 1.88 (d, *J* = 11.9 Hz, 2H), 2.20 (t, *J* = 11.0 Hz), 2.66-2.78 (m, 1H), 2.73-2.84 (m, 2H), 3.05 (d, *J* = 11.5 Hz, 2H), 3.30-3.46 (m, 2H), 4.08 (s, 3H), 7.11 (d, *J* = 9.0 Hz, 1H), 7.41 (d, *J* = 4.5 Hz, 1H), 8.19 (d, *J* = 9.0 Hz, 1H), 8.66 (d, *J* = 4.5 Hz, 1H) ppm;
**¹³C NMR (100 MHz, CDCl₃):** δ = 28.46, 35.83, 52.37, 53.72, 58.37, 77.23, 116.31, 124.24, 140.33, 140.97, 141.48, 146.64, 147.70, 161.43 ppm;
**IR (ATR):** v = 3271, 2941, 2811, 1614, 1590, 1503, 1489, 1438, 1398, 1334, 1125, 1079, 1018, 932, 853, 807, 751, 713, 615, 584, 536 cm⁻¹;
**HRMS (ESI+) *m*/*z*** for C₁₆H₂₂N₄O₁₄ ([M + H]⁺): calculated 287.1872, found 287.1875.

### Example 2 - Compound C1

*N*-benzyl-1-(2-(6-methoxy-1,5-naphthyridin-4-yl)ethyl)piperidin-4-amine

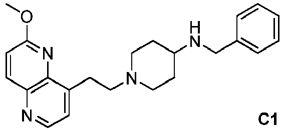

Starting from **I2** (0.2 g, 0.698 mmol, 1 equiv), benzaldehyde (0.092 mL, 0.908 mmol, 1.3 equiv) and NaCNBH₃ (0.175 g, 2.792 mmol, 4 equiv), compound **C1** was prepared according to the general procedure for reductive amination and purified with flash column chromatography using CHCl₃/MeOH (9:1) to afford yellow solid (0.064 g, 24.4%).
**Mp:** 47.7-50.1 °C;
**Rf:** 0.28 (DCM/MeOH 4:1), 0.14 (CHCl₃/MeOH 9:1);
**¹H NMR (400 MHz, CDCl₃): δ** = 1.48-1.58 (m, 2H), 1.98-2.01 (m, 2H), 2.19-2.25 (m, 2H), 2.57-2.62 (m, 1H), 2.80-2.84 (m, 2H), 3.07-3.10 (m, 2H), 3.39-3.43 (m, 2H), 3.86 (s, 2H), 4.10 (s, 3H), 7.13 *(d, J=* 8.8 Hz, 1H), 7.28-7.30 (m, 1H), 7.36 (d, *J=* 4.4 Hz, 4H), 7.43 (d, *J=* 4.4 Hz, 1H), 8.21 (d, *J=* 9.2 Hz, 1H), 8.68 (d, *J* = 4.4 Hz, 1H) ppm;
**¹³C (100 MHz, CDCl₃): δ** = 28.47, 32.80, 50.82, 52.35, 53.72, 54.09, 58.46, 77.24, 116.27, 124.24, 126.90, 128.06, 128.44, 140.34, 140.75, 140.99, 141.49, 146.75, 147.71, 161.40 ppm;
**IR (ATR): *v*** = 2942, 2817, 1611, 1590, 1489, 1452, 1397, 1361, 1334, 1301, 1256, 1183, 1107, 1075, 1018, 987, 849, 808, 747, 697, 649, 614, 537 cm⁻¹;
**MS (ESI+) *m*/*z*** = 377.3 (M + H)⁺;
**HRMS (ESI+) *m*/*z*** for C₂₃H₂₉ON₄ ([M + H]⁺): calculated 377.2336; found 377.2334.

### Example 3 - Compound C2

### (4-((1-(2-(6-methoxy-1,5-naphthyridin-4-yl)ethyl)piperidin-4-ylamino)methyl)phenyl)methanol

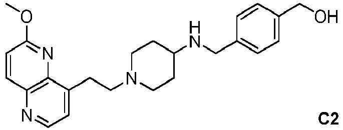

Starting from **I2** (0.2 g, 0.698 mmol, 1 equiv), 4-(hydroxymethyl)benzaldehyde (0.123 g, 0.908 mmol, 1.3 equiv) and NaCNBH₃ (0.175 g, 2.792 mmol, 4 equiv), compound **C2** was prepared according to the general procedure for reductive amination purified with flash column chromatography using CHCl₃/MeOH (9:1) to afford brown solid (0.122 g, 43.0%).
**Mp:** 73.4-76.7 °C;
**Rf:** 0.07 (DCM/MeOH 4:1), 0.03 (CHCl₃/MeOH 9:1);
**¹H NMR (400 MHz, CDCl₃): δ** = 1.46-1.55 (m, 2H), 1.95-1.98 (m, 2H), 2.17-2.22 (m, 2H), 2.53-2.59 (m, 1H), 2.78-2.82 (m, 2H), 3.04-3.07 (m, 2H), 3.37-3.41 (m, 2H), 3.83 (s, 2H), 4.07 (s, 3H), 4.69 (s, 2H), 7.11 (d, *J=* 8.8 Hz, 1H), 7.33 (s, 4H), 7.41 (d, *J* = 4.8 Hz, 1H), 8.18 (d, *J=* 8.8 Hz, 1H), 8.65 (d, *J*= 4.4 Hz, 1H) ppm;
**¹³C (100 MHz, CDCl₃): δ** = 28.39, 32.48, 50.39, 52.24, 53.67, 53.79, 58.35, 65.04, 77.21, 116.32, 124.25, 127.20, 128.33, 139.79, 140.32, 140.96, 141.46, 146.62, 147.66, 147.70, 161.43 ppm;
**IR (ATR): *v*** = 2938, 2821, 1611, 15920, 1503, 1487, 1446, 1397, 1333, 1257, 1174, 1104, 1073, 1050, 1017, 990, 930, 849, 805, 753, 613, 585 cm⁻¹;
**MS (ESI+) *m*/*****z*** = 407.3 (M + H)⁺;
**HRMS (ESI+) *m*/*****z*** for C₂₄H₃₁O₂N₄ ([M + H]⁺): calculated 407.2442, found 407.2440.

### Example 4 - Compound C3

### 4-((1-(2-(6-methoxy-1,5-naphthyridin-4-yl)ethyl)piperidin-4-ylamino)methyl)benzamide

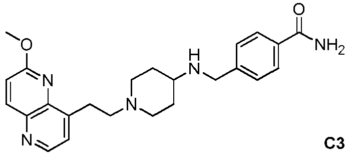

Starting from **I2** (0.2 g, 0.698 mmol, 1 equiv), 4-formylbenzamide (0.135 g, 0.908 mmol, 1.3 equiv) and NaCNBH₃ (0.175 g, 2.792 mmol, 4 equiv), compound **C3** was prepared according to the general procedure for reductive amination and purified with flash column chromatography using CHCl₃/MeOH (9:1) to afford yellow solid (0.033 g, 11.3%).
**Mp:** 116.1-122.1 °C;
**Rf:** 0.06 (DCM/MeOH 4:1), 0.02 (CHCl₃/MeOH 9:1);
**¹H NMR (400 MHz, CDCl₃): δ** = 1.44-1.54 (m, 2H), 1.94-1.96 (m, 2H), 2.14-2.20 (m, 2H), 2.51-2.56 (m, 1H), 2.77-2.81 (m, 2H), 3.04-3.07 (m, 2H), 3.36-3.40 (m, 2H), 3.90 (s, 2H), 4.07 (s, 3H), 5.59-6.17 (m, 2H), 7.11 (d, *J=* 8.8 Hz, 1H), 7.41-7.44 (m, 3H), 7.78 (d, *J=* 8.4 Hz, 2H), 8.18 (d, *J=* 9.2 Hz, 1H), 8.66 (d, *J* = 4.4 Hz, 1H) ppm;
**¹³C (100 MHz, DMSO): δ** = 27.49, 32.05, 49.33, 51.67, 53.27, 53.58, 57.85, 115.99, 124.46, 127.20, 127.41, 132.32, 140.24, 140.45, 140.81, 144.74, 146.15, 147.66, 160.80, 167.10 ppm;
**IR (ATR): *v*** = 3288, 3148, 2941, 2838, 1681, 1614, 1591, 1567, 1495, 1450, 1398, 1336, 1289, 1264, 1134, 1105, 1083, 1017, 990, 869, 851, 807, 782, 757, 691, 657, 631, 602, 586, 547 cm⁻¹;
**MS (ESI+) *m*/*****z*** = 420.3 (M + H)⁺;
**HRMS (ESI+) *m*/*z*** for C₂₄H₃₀O₂N₅ ([M + H]⁺): calculated 420.2394, found 420.2391.

### Example 5 - Compound C4

### N-(4-(dimethylamino)benzyl)-1-(2-(6-methoxy-1,5-naphthyridin-4-yl)ethyl)piperidin-4-amine

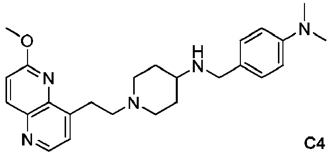

Starting from **I2** (0.2 g, 0.698 mmol, 1 equiv), 4-(dimethylamino)benzaldehyde (0.135 g, 0.908 mmol, 1.3 equiv) and NaCNBH₃ (0.175 g, 2.792 mmol, 4 equiv), compound **C4** was prepared according to the general procedure for reductive amination and purified with flash column chromatography using CHCl₃/MeOH (9:1) to afford yellow-orange solid (0.196 g, 66.9%).
**Mp:** 89.4-91.6 °C;
**Rf:** 0.07 (DCM/MeOH 4:1), 0.05 (CHCl₃/MeOH 9:1);
**¹H NMR (400 MHz, CDCl₃): δ** = 1.47-1.57 (m, 2H), 1.95-1.98 (m, 2H), 2.15-2.21 (m, 2H), 2.56-2.62 (m, 1H), 2.76-2.80 (m, 2H), 2.93 (s, 6H), 2.98-3.09 (m, 2H), 3.35-3.39 (m, 2H), 3.74 (s, 2H), 4.07 (s, 3H), 6.71 (d, *J* = 8.8 Hz, 2H), 7.11 (d, *J* = 8.8 Hz, 1H), 7.21 (d, *J* = 8.8 Hz, 2H), 7.41 (d, *J* = 4.4 Hz, 1H), 8.18 (d, *J* = 8.8 Hz, 1H), 8.66 (d, *J* = 4.4 Hz, 1H) ppm;
**¹³C (100 MHz, CDCl₃): δ** = 28.43, 32.33, 40.74, 49.97, 52.26, 53.72, 58.39, 77.23, 112.71, 116.26, 124.24, 129.19, 140.31, 140.96, 141.46, 146.71, 147.67, 149.87, 161.39 ppm;
**IR (ATR): *v*** = 2920, 2802, 1611, 1593, 1523, 1488, 1397, 2335, 1262, 1217, 1191, 1166, 1146, 1104, 1063, 1018, 958, 934, 856, 837, 809, 755, 712, 696, 647, 586, 566 cm⁻¹;
**MS (ESI+) *m*/*z*** = 420.3 (M + H)⁺;
**HRMS (ESI+) *m*/*z*** for C₂₅H₃₄ON₅ ([M + H]⁺): calculated 420.2758, found 420.2754.

### Example 6 - Compound C5

### N (4-fluorobenzyl)-1-(2-(6-methoxy-1,5-naphthyridin-4-yl)ethyl)piperidin-4-amine

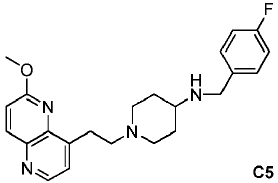

Starting from **I2** (0.233 g, 0.814 mmol, 1 equiv), 4-fluorobenzaldehyde (0.09 mL, 0.853 mmol, 1.05 equiv) and NaCNBH₃ (0.179 g, 2.849 mmol, 3.5 equiv), compound **C5** was prepared according to the general procedure for reductive amination and purified with flash column chromatography using CHCl₃/MeOH (9:1 + 1% Et₃N) to afford light brown solid (0.233 g, 69.2%).
**Mp** = 55-60 °C;
**Rf:** 0.2 (DCM/MeOH 4:1 + 1 % Et₃N);
**¹H NMR (400 MHz, CDCl₃):** δ = 1.44-1.55 (m, 2H), 1.95 (d, *J* = 12.6 Hz, 2H), 2.18 (t, *J* = 10.5 Hz, 2H), 2.46-2.62 (m, 1H), 2.74-2.86 (m, 2H), 3.05 (d, *J* = 11.8 Hz, 2H), 3.31-3.44 (m, 2H), 3.80 (s, 2H), 4.07 (s, 3H), 6.97-7.04 (m, 2H), 7.11 (d, *J* = 9.0 Hz, 1H), 7.27-7.33 (m, 2H), 7.41 (d, *J* = 4.5 Hz, 1H), 8.18 (d, *J* = 9.0 Hz, 1H), 8.66 (d, *J* = 4.5 Hz, 1H) ppm;
**¹³C NMR (100 MHz, CDCl₃):** δ = 28.43, 32.71, 50.07, 52.32, 53.71, 58.41, 77.24, 115.20 (d, *J* = 21.2 Hz), 116.29, 124.25, 129.54 (d, *J* = 8.0 Hz), 136.41, 140.35, 140.97, 141.50, 146.67, 147.71, 161.42, 161.88 (d, *J* = 244.6 Hz) ppm;
**IR (ATR): *v*** = 3035, 2935, 2811, 1611, 1592, 1505, 1489, 1468, 1441, 1398, 1369, 1334, 1299, 1263, 1215, 1106, 1077, 1017, 932, 850, 812, 790, 751, 712, 696, 647, 607, 584, 564 cm⁻¹;
**HRMS (ESI+) *m*/*z*** for C₂₃H₂₇FN₄O ([M + H]⁺): calculated 395.2242; found 395.2238.

### Example 7 - Compound C6

### N-(4-chlorobenzyl)-1-(2-(6-methoxy-1,5-naphthyridin-4-yl)ethyl)piperidin-4-amine

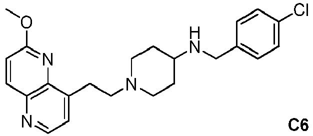

Starting from **I2** (0.2 g, 0.698 mmol, 1 equiv), 4-chlorobenzaldehyde (0.128 g, 0.908 mmol, 1.3 equiv) and NaCNBH₃ (0.175 g, 2.792 mmol, 4 equiv), compound **C6** was prepared according to the general procedure for reductive amination and purified with flash column chromatography using CHCl₃/MeOH (9:1) to afford orange-brown solid (0.220 g, 76.7%).
**Mp:** 67.6-68.4 °C;
**Rf:** 0.44 (DCM/MeOH 4:1), 0.32 (CHCl₃/MeOH 4:1);
**¹H NMR (400 MHz, CDCl₃): δ** = 1.43-1.53 (m, 2H), 1.93-1.96 (m, 2H), 2.15-2.20 (m, 2H), 2.50-2.55 (m, 1H), 2.77-2.81 (m, 2H), 3.03-3.06 (m, 2H), 3.36-3.40 (m, 2H), 3.80 (s, 2H), 4.07 (s, 3H), 7.11 (d, *J* = 8.8 Hz, 1H), 7.26-7.31 (m, 4H), 7.41 (d, *J* = 4.4 Hz, 1H), 8.18 (d, *J* = 8.8 Hz, 1H), 8.65 (d, *J* = 4.4 Hz, 1H) ppm;
¹**³C (100 MHz, CDCl₃): δ** = 28.44, 32.69, 50.06, 52.29, 53.71, 54.04, 58.40, 77.25, 116.30, 124.74, 128.53, 129. 39, 132.56, 139.19, 140.32, 140.96, 141.47, 146.65, 147.69, 161.42 ppm;
**IR (ATR): *v*** = 3223, 2945, 2827, 1490, 1262, 1092, 857, 810, 757, 641, 603 cm⁻¹;
**MS (ESI+) *m*/*****z*** = 411.3 (M + H)⁺;
**HRMS (ESI+) *m*/*z*** for C₂₃H₂₈ON₄Cl ([M + H]⁺): calculated 411.1946, found 411.1930.

### Example 8 - Compound C7

### N (4-bromobenzyl)-1-(2-(6-methoxy-1,5-naphthyridin-4-yl)ethyl)piperidin-4-amine

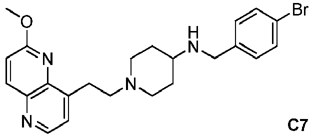

Starting from **I2** (0.2 g, 0.698 mmol, 1 equiv), 4-bromobenzaldehyde (0.168 g, 0.908 mmol, 1.3 equiv) and NaCNBH₃ (0.175 g, 2.792 mmol, 4 equiv), compound **C7** was prepared according to the general procedure for reductive amination and purified with flash column chromatography using CHCl₃/MeOH (9:1) to afford yellow-orange solid (0.293 g, 92.2%).
**Mp:** 74.7-75.8 °C;
**Rf:** 0.55 (DCM/MeOH 4:1), 0.29 (CHCl₃/MeOH 9:1);
**¹H NMR (400 MHz, CDCl₃)**: **δ** = 1.43-1.52 (m, 2H), 1.93-1.96 (m, 2H), 2.15-2.20 (m, 2H), 2.50-2.55 (m, 1H), 2.77-2.81 (m, 2H), 3.03-3.06 (m, 2H), 3.36-3.40 (m, 2H), 3.79 (s, 2H), 4.07 (s, 3H), 7.11 (d, *J* = 8.8 Hz, 1H), 7.22 (d, *J* = 6.32, 2H), 7.40-7.45 (m, 3H), 8.18 (d, *J* = 8.8 Hz, 1H), 8.65 (d, *J* = 4.4 Hz, 1H) ppm;
**¹³C (100 MHz, CDCl₃): δ** = 28.44, 32.69, 50.10, 52.29, 53.71, 54.03, 58.40, 77.25, 116.30, 120.63, 124.74, 129.77, 131.48, 139.72, 140.32, 140.97, 141.46, 146.66, 147.68, 161.42 ppm;
**IR (ATR): v** = 3224, 2942, 2826, 1589, 1489, 1402, 1335, 1263, 1092, 1006, 856, 810, 757, 656, 634, 598, 545, 521 cm⁻¹;
**MS (ESI+) *m*/*z*** = 455.1 (M + H)⁺;
**HRMS (ESI+) *m*/*z*** for C₂₃H₂₈ON₄Br ([M + H]⁺): calculated 455.1441, found 455.1424.

### Example 9 - Compound C8

### N-(4-iodobenzyl)-1-(2-(6-methoxy-1,5-naphthyridin-4-yl)ethyl)piperidin-4-amine

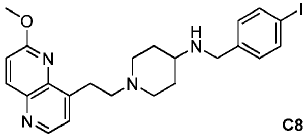

Starting from **I2** (0.2 g, 0.698 mmol, 1 equiv), 4-iodobenzaldehyde (0.210 g, 0.908 mmol, 1.3 equiv) and NaCNBH₃ (0.175 g, 2.792 mmol, 4 equiv), compound **C8** was prepared according to the general procedure for reductive amination and purified with flash column chromatography using CHCl₃/MeOH (9:1) to afford brown-yellow solid (0.285 g, 81.3%).
**Mp:** 80.8-82.7 °C;
**Rf:** 0.40 (DCM/MeOH 4:1), 0.17 (CHCl₃/MeOH 9:1);
**¹H NMR (400 MHz, CDCl₃): δ** = 1.43-1.52 (m, 2H), 1.92-1.95 (m, 2H), 2.14-2.20 (m, 2H), 2.50-2.55 (m, 1H), 2.77-2.81 (m, 2H), 3.03-3.06 (m, 2H), 3.36-3.40 (m, 2H), 3.78 (s, 2H), 4.07 (s, 3H), 7.08-7.12 (m, 3H), 7.41 (d, *J=* 4.4 Hz, 1H), 7.41 (d, *J=* 4.4 Hz, 1H), 7.64 (d, *J=* 8.4 Hz, 2H), 8.18 (d, *J* = 8.8 Hz, 1H), 8.66 (d, *J=* 4.4 Hz, 1H) ppm;
**¹³C (100 MHz, DMSO-d6): δ** = 28.42, 32.66, 50.17, 52.27, 53.72, 58.38, 77.24, 92.10, 116.30, 124.26, 130.07, 137.46, 140.36, 140.44, 140.96, 141.50, 146.60, 147.72, 161.43 ppm;
**IR (ATR): *v*** = 2921, 2791, 1612, 1586, 1502, 1487, 1398, 1333, 1260, 1142, 1120, 1095, 1076, 1007, 930, 886, 855, 800, 737, 706, 627, 583, 538 cm⁻¹;
**MS (ESI+) *m*/*****z*** = 503.2 (M + H)⁺;
**HRMS (ESI+) *m*/*****z*** for C₂₃H₂₈ON₄ ([M + H]⁺): calculated 503.1302, found 503.1298.

### Example 10 - Compound C9

### N-(4-(difluoromethoxy)-3-methoxybenzyl)-1-(2-(6-methoxy-1,5-naphthyridin-4-yl)ethyl)piperidin-4-amine

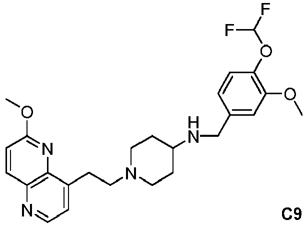

Starting from **I2** (0.194 g, 0.677 mmol, 1 equiv), 4-(difluoromethoxy)-3-methoxybenzaldehyde (0.120 mL, 0.748 mmol, 1.1 equiv) and NaCNBH₃ (0.149 g, 2.371 mmol, 3.5 equiv), compound **C9** was prepared according to the general procedure for reductive amination and purified with flash column chromatography using CHCl₃/MeOH (9:1 + 1% Et₃N) to afford red-brown solid (0.194 g, 54.9%).
**Mp** = 85-93 °C;
**Rf:** 0.19 (DCM/MeOH 4:1 + 1 % Et₃N);
**¹H NMR (400 MHz, CDCl₃):** δ = 1.44-1.55 (m, 2H), 1.96 (d, *J* = 11.6 Hz, 2H), 2.19 (t, *J* = 10.5 Hz, 2H), 2.48-2.59 (m, 1H), 2.72-2.84 (m, 2H), 3.06 (d, *J* = 11.7 Hz, 2H), 3.34-3.41 (m, 2H), 3.81 (s, 2H), 3.89 (s, 3H), 4.07 (s, 3H), 6.53 (t, *J* = 75.4 Hz, 1H), 6.88 (dd, *J₁* = 8.1 Hz, *J₂* = 1.8 Hz, 1H), 7.01 (d, *J* = 1.6 Hz, 1H), 7.10 (d, *J* = 8.4 Hz, 1H), 7.11 (d, *J* = 8.8 Hz, 1H), 7.41 (d, *J* = 4.5 Hz, 1H), 8.18 (d, *J* = 9.0 Hz, 1H), 8.66 (d, *J* = 4.5 Hz, 1H) ppm;
**¹³C NMR (100 MHz, CDCl₃):** δ = 28.45, 32.79, 50.51, 52.36, 53.71, 55.91, 58.41, 77.23, 112.34, 116.25 (t, *J* = 259.5 Hz), 116.29, 120.21, 122.22, 124.25, 138.73, 139.72, 140.37, 140.97, 141.50, 146.62, 147.72, 151.08, 161.42 ppm;
**IR (ATR): *v*** = 2937, 2832, 1610, 1592, 1507, 1490, 1465, 1419, 1398, 1374, 1334, 1264, 1212, 1191, 1079, 1034, 1015, 853, 811, 787, 753, 732, 586 cm⁻¹;
**HRMS (ESI+) *m*/*z*** for C₂₅H₃₀F₂N₄O₃ ([M + H]⁺): calculated 473.2359; found 473.2354.

### Example 11 - Compound C10

### 2-(difluoromethoxy)-5 -((1 -(2-(6-methoxy-1,5-naphthyridin-4-yl)ethyl)piperidin-4-ylamino)methyl)phenol

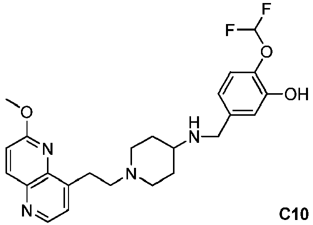

Starting from **I2** (0.171 g, 0.597 mmol, 1 equiv), 4-(difluoromethoxy)-3-hydroxybenzaldehyde (0.138 g, 0.734 mmol, 1.2 equiv) and NaCNBH₃ (0.131 g, 2.085 mmol, 3.5 equiv), compound **C10** was prepared according to the general procedure for reductive amination and purified with flash column chromatography using CHCl₃/MeOH (9:1 + 1% Et₃N) to afford light brown solid (0.171 g, 50.9%).
**Mp** =99-104 °C;
**Rf:** 0.09 (DCM/MeOH 4:1 + 1 % Et₃N);
**¹H NMR (400 MHz, CDCl₃):** δ = 1.50-1.61 (m, 2H), 1.99 (d, *J* = 10.9 Hz, 2H), 2.28 (t, *J* = 10.7 Hz, 2H), 2.60-2.68 (m, 1H), 2.83-2.89 (m, 2H), 3.12 (d, *J* = 11.7 Hz, 2H), 3.38-3.43 (m, 2H), 3.75 (s, 2H), 4.07 (s, 3H), 6.51 (t, *J* = 74.3 Hz, 1H), 6.79 (dd, *J*₁ = 8.2 Hz, *J*₂ =1.9 Hz, 1H), 6.97 (d, *J* = 1.9 Hz, 1H), 7.02 (d, *J* = 8.2 Hz, 1H), 7.11 (d, *J* = 9.0 Hz, 1H), 7.42 (d, *J* = 4.5 Hz, 1H), 8.19 (d, *J* = 9.0 Hz, 1H), 8.66 (d, *J* = 4.5 Hz, 1H) ppm;
**¹³C NMR (100 MHz, CDCl₃):** δ = 28.12, 31.72, 49.94, 51.96, 53.77, 58.06, 77.23*, 116.38 (t, *J* = 260.8 Hz), 116.46, 117.32, 119.91, 120.53, 124.34, 137.80, 138.54, 140.22, 140.89, 141.38, 146.10, 147.61, 148.08, 161.53 ppm;
**IR (ATR): v** = 2944, 2338, 1612, 1595, 1490, 1438, 1399, 1334, 1297, 1261, 1113, 1076, 1049, 1014, 851, 809, 782, 752, 714, 674, 641, 586 cm⁻¹;
**HRMS (ESI+) *m*/*z*** for C₂₄H₂₈F₂N₄O₃ ([M + H]⁺): calculated 459.2202; found 459.2196.

### Example 12 - Compound C11

### N-((1-allyl-1H-pyrazol-4-yl)methyl)-1-(2-(6-methoxy-1,5-naphthyridin-4-yl)ethyl)piperidin-4-amine

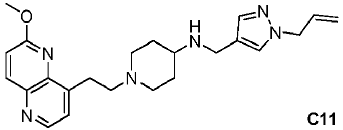

Starting from **I2** (0.2 g, 0.698 mmol, 1 equiv), 1-allyl-1H-pyrazole-4-carbaldehyde (0.112 g, 0.823 mmol, 1.2 equiv) and NaCNBH₃ (0.166 g, 2.642 mmol, 3.8 equiv), compound **C11** was prepared according to the general procedure for reductive amination and purified with flash column chromatography using CHCl₃/MeOH (9:1 + 1% Et₃N) to afford dark red viscous liquid (0.200 g, 59.8%).
**Rf:** 0.16 (DCM/MeOH 4:1 + 1 % Et₃N);
**¹H NMR (400 MHz, CDCl₃):** δ = 1.50-1.61 (m, 2H), 2.00 (d, *J* = 11.1 Hz, 2H), 2.30 (t, *J* = 12.9 Hz, 2H), 2.60-2.71 (m, 1H), 2.83-2.91 (m, 2H), 3.11 (d, *J* = 11.8 Hz, 2H), 3.37-3.46 (m, 2H), 3.74 (s, 2H), 4.08 (s, 3H), 4.71 (m, 2H), 5.19-5.31 (m, 2H), 6.01 (m, 1H), 7.12 (d, *J* = 9.0 Hz, 1H), 7.43 (s, 3H), 8.19 (d, *J* = 9.0 Hz, 1H), 8.66 (d, *J* = 4.5 Hz, 1H) ppm;
**¹³C NMR (100 MHz, CDCl₃):** δ = 28.19, 31.71, 40.57, 51.98, 53.77, 54.79, 58.15, 77.23, 116.39, 118.78, 124.32, 128.19, 132.84, 139.02, 140.38, 140.90, 141.52, 147.74, 161.51ppm;
**IR (ATR): *v*** = 2938, 2805, 2324, 2167, 1612, 1589, 1489, 1439, 1398, 1372, 1333, 1260, 1119, 1077, 1018, 990, 928, 853, 807, 731, 642, 585 cm⁻¹;
**HRMS (ESI+) *m*/*z*** for C₂₃H₃₀N₆O ([M + H]⁺): calculated 407.2554; found 407.2547.

### Example 13 - Biological testing

### 1) Bacterial DNA gyrase inhibition determination

### Experimental method:

The concentrations of the compounds needed to inhibit enzyme residual activity by half (IC₅₀ values) were determined by Gyrase Supercoiling High Throughput Plate assay kit, purchased from Inspiralis (Norwich, UK) following their protocol. The method involved fluorescence measurements of enzyme activity at compound dilution series with seven concentrations, performed on black streptavidin-coated 96-wells microtiter plates. All example compounds were assayed against isolated Gram-positive *Staphylococcus aureus* and Gram-negative *Escherichia coli* DNA gyrase enzyme, respectively, supplied as separate assay kits.

### Results:

The results of IC₅₀ values are given in Table 1 and correspond to the mean of two to four independent measurements. Ciprofloxacin was used as a reference compound with IC₅₀ 93.65 µM and 0.44 µM for S. *aureus* and *E. coli,* respectively.

### 2) Human topoisomerase II Alpha inhibition determination

### Experimental method:

The selectivity of example compounds for bacterial DNA gyrase over the orthologous human topoisomerase IIα was determined by Human topoisomerase II Alpha Relaxation High Throughput Plate assay kit, purchased from Inspiralis (Norwich, UK) following their protocol. The method involved fluorescence measurements of enzyme activity at 10 µM compound concentration, performed on black streptavidin-coated 96-wells microtiter plates.

### Results:

Selectivity of all example compounds was confirmed and the mean percentages of human topoisomerase IIα residual activity (% RA) of two independent measurements are given in Table 1.

**Table 1. In vitro bacterial DNA gyrase inhibitory activity and human topo IIα selectivity.**

| **Compound** | **IC₅₀ [µM]** | | **Human Topo IIα [% RA]** |
|---|---|---|---|
| | ***S. aureus*** | ***E. coli*** | |
| **C1** | 1.02 | 40.60 | 96.97 ± 9.58 |
| **C2** | 4.39 | 56.56 | 97.73 ± 1.08 |
| **C3** | 1.82 | > 100 | 97.80 ± 2.21 |
| **C4** | 0.067 | 11.89 | 94.19 ± 1.33 |
| **C5** | 0.55 | 16.95 | 100.81 ± 11.22 |
| **C6** | 0.035 | 1.71 | 101.98 ± 0.84 |
| **C7** | 0.007 | 0.57 | 98.25 ± 2.72 |
| **C8** | 0.011 | 0.28 | 88.26 ± 3.33 |
| **C9** | 0.35 | 3.90 | 96.36 ± 2.30 |
| **C10** | 0.33 | 4.41 | 94.80 ± 0 |
| **C11** | 1.10 | 16.70 | 95.69 ± 6.42 |

### 3) Minimal inhibitory concentration (MIC) determination

### Experimental method:

Minimal inhibitory concentrations were determined by the broth microdilution method in 96-wells plate format following the Clinical and Laboratory Standards Institute (CLSI) guidelines given in "Methods for Dilution Antimicrobial Susceptibility Tests for Bacteria That Grow Aerobically, Approved Standard, 10th Edition, CLSI document M07-A10, Wayne, PA: Clinical and Laboratory Standards Institute, USA, 2015" and European Committee on Antimicrobial Susceptibility Testing (EUCAST) recommendations given in "The European Committee on Antimicrobial Susceptibility Testing, Breakpoint tables for interpretation of MICs and zone diameters, Version 5.0, 2015". All exemplified compounds were tested on several Gram-positive (*S*. *aureus,* methicillin-resistant *Staphylococcus aureus* (MRSA), methicillin-resistant *Staphylococcus schleiferi* subspecies *coagulans,* methicillin-resistant *Staphylococcus pseudintermedius* (S-48, S-84, S-35, S-52, S-40 and S-51), *Streptococcus agalactiae* and *Enterococcus faecalis*) and Gram-negative bacteria (E. *coli, Salmonella* Alachua and *Pseudomonas aeruginosa*)*.*

### Results:

The MICs were visually determined as the lowest compound concentration inhibiting bacterial growth after 20 hours of incubation time. Tetracycline was used as a reference compound, showing MIC of 0.5 µg/mL and 1 µg/mL for S. *aureus* and *E. coli,* respectively. MIC values are represented in Table 2.

**Table 2. Antibacterial activity.**

| **Compoun d** | **MIC [µg/mL]** | | | | | | | | | | | | | | | | |
|---|---|---|---|---|---|---|---|---|---|---|---|---|---|---|---|---|---|
| | **S. *aureu s*** | ***E. coli*** | **MRS A** | **MRSS** | **MRS P S-84** | **MRS P S-48** | **MRS P S-35** | **MRS P S-52** | **MRSP S-40** | **MRS P S-51** | **S. *agalactia e* RDK 047** | **S. Alachu a RDK 030c** | ***E. faecali s* DRK 057** | **P. *aeruginos a* RDK 184** | **S. *pneumonia e*** | **A. *baumann ii* 8C6** | ***C*. *jejun i* RDK -135** |
| **C1** | 4 | 64 | 8 | 0.25 | 4 | 4 | 4 | 16 | 4 | 4 | 16 | >128 | 16 | >128 | 2 | 64 | 32 |
| **C2** | 4 | 128 | 4 | 0.25 | 4 | 4 | 4 | 8 | 4 | 4 | 8 | >128 | 8 | >128 | 2 | 128 | 32 |
| **C3** | 2 | >12 | 4 | 0.25 | 4 | 4 | 4 | 8 | 4 | 4 | >128 | >128 | 128 | >128 | 16 | 128 | 128 |
| | | 8 | | | | | | | | | | | | | | | |
| **C4** | 0.125 | 32 | 0.025 | 0.015 | 0.125 | 0.25 | 0.25 | 0.5 | 0.125 | 0.25 | 0.25 | 128 | 1 | >128 | 0.25 | 8 | 4 |
| **C5** | 1 | 32 | 2 | 0.125 | 2 | 2 | 2 | 8 | 2 | 2 | 8 | 128 | 8 | >128 | 2 | 32 | 1 |
| **C6** | 0.125 | 4 | 0.25 | ≤0.007 | 0.25 | 0.25 | 0.25 | 0.5 | 0.25 | 0.25 | 1 | 32 | 1 | >128 | 0.5 | 4 | 2 |
| | | | | 8 | | | | | | | | | | | | | |
| **C7** | 0.031 | 2 | 0.062 | ≤0.007 | 0.062 | 0.062 | 0.062 | 0.125 | 0.031 | 0.062 | 0.5 | 16 | 1 | 128 | 0.125 | 1 | 0.5 |
| | | | | 8 | | | | | | | | | | | | | |
| **C8** | 0.007 | 2 | 0.015 | 0.002 | 0.015 | 0.015 | 0.15 | 0.062 | ≤0.007 | 0.007 | 0.125 | 8 | 1 | 32 | 0.015 | 0.25 | 0.015 |
| | 8 | | | | | | | | 8 | 8 | | | | | | | |
| **C9** | 1 | 32 | 2 | 0.125 | 2 | 2 | 2 | 4 | 2 | 2 | <1 | 128 | 16 | >128 | 2 | 8 | 2 |
| **C10** | 0.5 | 8 | 1 | 0.25 | 0.25 | 0.25 | 0.5 | 2 | 0.25 | 0.25 | 2 | 64 | 4 | >128 | 1 | 4 | 0.016 |
| **C11** | 2 | 16 | 2 | 0.125 | <1 | 2 | 2 | 4 | 1 | 2 | 4 | 128 | 8 | >128 | 1 | 32 | 8 |

### 4) Cytotoxicity assessment

### Experimental method:

Cytotoxicity was assessed in assay measuring metabolic cell activity performed on HepG2 (ATCC) and HUVEC (ATCC) cell lines according to the protocol of CellTiter 96^{®} AQueous One Solution Cell Proliferation Assay 2018, Promega, USA, 2001. The method involved absorbance measurements after the treatment of cells with compounds at concentrations 1 µM and 50 µM.

### Results:

The results are given as the mean percentage of metabolic activity of treated cells from two independent experiments and are represented in Table 3.

**Table 3. Evaluation of the compound toxicity to the cells.**

| **Compound** | **HUVEC** | | **HepG2** | |
|---|---|---|---|---|
| | **1 µM** | **50 µM** | **1 µM** | **50 µM** |
| **C1** | 92.9 ± 16.6 | 63.4 ± 11.3 | 107.1 ± 7.2 | 1.4 ± 0.3 |
| **C2** | 97.7 ± 9.4 | 63.1 ± 2.1 | 100.2 ± 1.5 | 24.1 ± 0.9 |
| **C3** | 105.8 ± 3.5 | 66.1 ± 3.5 | 94.0 ± 0.8 | 48.3 ± 1.0 |
| **C4** | 105.9 ± 0.9 | 0.1 ± 0.6 | 102.5 ± 2.3 | 0.8 ± 1.0 |
| **C5** | 105.2 ± 15.1 | 75.6 ± 12.0 | 98.0 ± 3.9 | -1.3 ± 2.2 |
| **C6** | 86.1 ± 17.1 | -0.6 ± 0.4 | 84.5 ± 11.1 | 0.9 ± 0.1 |
| **C7** | 92.0 ± 4.5 | -0.5 ± 0.2 | 84.3 ± 8.9 | -0.1 ± 0.3 |
| **C8** | 108.0 ± 6.6 | -0.3 ± 0.5 | 102.6 ± 2.3 | 0.5 ± 0.3 |
| **C9** | 89.6 ± 3.8 | 0.2 ± 1.0 | 89.7 ± 3.8 | -0.3 ± 2.1 |
| **C10** | 96.4 ± 9.1 | 2.5 ± 0.3 | 100.5 ± 4.8 | -0.4 ± 1.6 |
| **C11** | 88.6 ± 21.2 | 84.2 ± 19.5 | 108.5 ± 6.3 | 77.0 ± 2.4 |

## Claims

1. A compound of Formula (I): 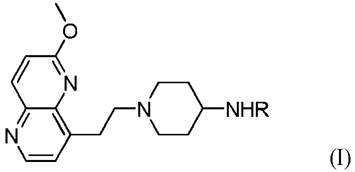 wherein R is a -CH₂-R₁ group with R₁ being a substituted or unsubstituted monocyclic aryl or a substituted or unsubstituted monocyclic aromatic heterocycle;
or a corresponding salt, solvate, hydrate or oxide thereof.

2. Compound according to claim 1, wherein R₁ is an unsubstituted monocyclic aryl, preferably unsubstituted phenyl.

3. Compound according to claim 1, wherein R₁ is a monocyclic aryl, preferably phenyl, substituted with one or more substituents selected from OH, halogen, CR₃R₄R₅, NR₃R₄, OCR₃R₄R₅ and COR₆; wherein R₃, R₄ and R₅ are independently selected from H, OH, halogen and (C₁₋₆)alkyl, and R₆ is selected from H, OH, halogen, (C₁₋₆)alkyl and NR₇R₈, with R₇ and R₈ being independently selected from H, halogen and (C₁₋₆)alkyl.

4. Compound according to claim 1, wherein R₁ a monocyclic aryl, preferably phenyl, substituted with one or more substituents selected from OH, halogen, CHzOH, C(O)NH₂, NH₂, N(CH₃)₂, OCH₃ and OCHR₉R₁₀ with R₉ and R₁₀ being each halogen.

5. Compound according to claim 4, wherein R₉ and R₁₀ are each fluoro.

6. Compound according to any one of claims 3 to 5, wherein the monocyclic aryl is mono-substituted, preferably 4-mono-substituted, or wherein the monocyclic aryl is di-substituted, preferably 3,4-di-substituted.

7. Compound according to claim 1, wherein R₁ is an unsubstituted monocyclic aromatic heterocycle, preferably an unsubstituted 5- or 6- membered monocyclic aromatic heterocycle.

8. Compound according to claim 1, wherein R₁ is a substituted monocyclic aromatic heterocycle, preferably a substituted 5- or 6- membered aromatic heterocycle, substituted with one or more substituents selected from (C₂₋₆)alkenyl, (C₄₋₆)dienyl, (C₂₋₆)alkynyl, and (C₄₋₆)diynyl.

9. Compound according to claim 8, wherein the heterocycle has one, two or three N heteroatoms and wherein at least one N heteroatom is substituted with one or more substituents selected from (C2-6)alkenyl, (C4-6)dienyl, (C2-6)alkynyl, and (C4-6)diynyl or wherein the heterocycle has one, two or three N heteroatoms and wherein at least one N heteroatom is substituted with one or more substituents selected from selected from ethenyl, propenyl, butenyl, pentenyl and hexenyl, preferably from vinyl, 2-propenyl, 3-butenyl, 4-pentenyl and 5-hexenyl.

10. Compound according to claim 8 or 9, wherein the monocyclic aromatic heterocycle is mono-substituted.

11. Compound according to any one of claims 7 to 10 wherein the monocyclic aromatic heterocycle is an azole, preferably the monocyclic aromatic heterocycle is a pyrazole, imidazole, 1,2,3-triazole or 1,2,4-triazole, more preferably the monocyclic aromatic heterocycle is a pyrazole.

12. Compound according to any one of claims 7 to 11, wherein the monocyclic aromatic heterocycle is 1*H*-pyrazol-4-yl substituted at position 1 with (C₂₋₆)alkenyl, preferably 1H-pyrazol-4-yl substituted at position 1 with allyl.

13. Compound according to claim 1, which is selected from the following list:
*N*-benzyl-1-(2-(6-methoxy-1,5-naphthyridin-4-yl)ethyl)piperidin-4-amine;
(4-((1-(2-(6-methoxy-1,5-naphthyridin-4-yl)ethyl)piperidin-4-ylamino)methyl)phenyl)methanol;
4-((1-(2-(6-methoxy-1,5-naphthyridin-4-yl)ethyl)piperidin-4-ylamino)methyl)benzamide;
*N*-(4-(dimethylamino)benzyl)-1-(2-(6-methoxy-1,5-naphthyridin-4-yl)ethyl)piperidin-4-amine;
*N*-(4-fluorobenzyl)-1-(2-(6-methoxy-1,5-naphthyridin-4-yl)ethyl)piperidin-4-amine;
*N*-(4-chlorobenzyl)-1-(2-(6-methoxy-1,5-naphthyridin-4-yl)ethyl)piperidin-4-amine;
*N*-(4-bromobenzyl)-1-(2-(6-methoxy-1,5-naphthyridin-4-yl)ethyl)piperidin-4-amine;
*N-*(4-iodobenzyl)-1-(2-(6-methoxy-1,5-naphthyridin-4-yl)ethyl)piperidin-4-amine;
*N*-(4-(difluoromethoxy)-3-methoxybenzyl)- 1-(2-(6-methoxy- 1,5-naphthyn*din-4-yl)ethyl)piperidin-4-amine;
2-(difluoromethoxy)-5 -((1-(2-(6-methoxy-1,5 -naphthyridin-4-yl)ethyl)piperidin-4-ylamino)methyl)phenol; and
*N*-((1-allyl-1*H-*pyrazol-4-yl)methyl)-1-(2-(6-methoxy-1,5 -naphthyridin-4-yl)ethyl)piperidin-4-amine.

14. A pharmaceutical composition comprising a compound as defined in any one of claims 1 to 13 or a pharmaceutically acceptable salt thereof, and a pharmaceutically acceptable carrier, adjuvant or vehicle.

15. A compound as defined in any one of claims 1 to 13 for use as a medicament, preferably for use as a medicament for the treatment of a bacterial infection.

## Patentansprüche

1. Verbindung der Formel (I): 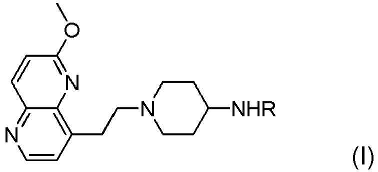 wobei R eine -CH₂-R₁-Gruppe ist, wobei R₁ ein substituiertes oder unsubstituiertes monocyclisches Aryl oder ein substituiertes oder unsubstituiertes monocyclisches aromatisches Heterocyclus ist;
oder ein entsprechendes Salz, Solvat, Hydrat oder Oxid davon.

2. Verbindung nach Anspruch 1, wobei R₁ ein unsubstituiertes monocyclisches Aryl, vorzugsweise ein unsubstituiertes Phenyl, ist.

3. Verbindung nach Anspruch 1, wobei R₁ ein monocyclisches Aryl, vorzugsweise Phenyl, mit einem oder mehreren Substituenten ausgewählt aus OH, Halogen, CR₃R₄R₅, NR₃R₄, OCR₃R₄R₅ und COR₆, ist; wobei R₃, R₄ und R₅ unabhängig aus H, OH, Halogen und (C₁₋₆)Alkyl ausgewählt sind, und R₆ aus H, OH, Halogen, (C₁₋₆)Alkyl und NR₇R₈ ausgewählt ist, wobei R₇ und R₈ unabhängig aus H, Halogen und (C₁₋₆)Alkyl ausgewählt sind.

4. Verbindung nach Anspruch 1, wobei R₁ ein monocyclisches Aryl, vorzugsweise Phenyl, ist, das mit einem oder mehreren Substituenten auswählt aus OH, Halogen, CH₂OH, C(O)NH₂, NH₂, N(CH₃)₂, OCH₃ und OCHR₉R₁₀ substituiert ist, wobei Rg und R₁₀ jeweils Halogen sind.

5. Verbindung nach Anspruch 4, wobei Rg und R₁₀ jeweils Fluor sind.

6. Verbindung nach einem der Ansprüche 3 bis 5, wobei das monocyclische Aryl mono-substituiert ist, vorzugsweise 4-mono-substituiert ist, oder wobei das monocyclische Aryl di-substituiert ist, vorzugsweise 3,4-di-substituiert ist.

7. Verbindung nach Anspruch 1, wobei R₁ ein unsubstituierter monocyclischer aromatischer Heterocyclus ist, vorzugsweise ein unsubstituierter 5- oder 6-gliedriger monocyclischer aromatischer Heterocyclus ist.

8. Verbindung nach Anspruch 1, wobei R₁ ein substituierter monocyclischer aromatischer Heterocyclus, vorzugsweise ein substituierter 5- oder 6-gliedriger aromatischer Heterocyclus ist, der mit einem oder mehreren Substituenten ausgewählt aus (C₂₋₆)Alkenyl, (C₄₋₆)Dienyl, (C₂₋₆)Alkynyl und (C₄₋₆)Diynyl substituiert ist.

9. Verbindung nach Anspruch 8, wobei der Heterocyclus ein, zwei oder drei N-Heteroatome aufweist, und wobei mindestens ein N-Heteroatom mit einem oder mehreren Substituenten ausgewählt aus (C2-6)Alkenyl, (C4-6)Dienyl, (C2-6)Alkynyl und (C4-6)Diynyl substituiert ist, oder wobei der Heterocyklus ein, zwei oder drei N-Heteroatome aufweist, und wobei mindestens ein N-Heteroatom mit einem oder mehreren Substituenten ausgewählt aus Ethenyl, Propenyl, Butenyl, Pentenyl und Hexenyl, vorzugsweise aus Vinyl, 2-Propenyl, 3-Butenyl, 4-Pentenyl und 5-hexenyl substituiert ist.

10. Verbindung nach Anspruch 8 oder 9, wobei der monocyclische aromatische Heterocyclus mono-substituiert ist.

11. Verbindung nach einem der Ansprüche 7 bis 10, wobei der monocyclische aromatische Heterocyclus ein Azol ist, vorzugsweise der monocyclische aromatische Heterocyclus ein Pyrazol, Imidazol, 1,2,3-Triazol oder 1,2,4-Triazol ist, eher bevorzugt der monocyclische aromatische Heterocyclus ein Pyrazol ist.

12. Verbindung nach einem der Ansprüche 7 bis 11, wobei der monocyclische aromatische Heterocyclus 1*H*-pyrazol-4-yl ist, das an Position 1 mit (C₂₋₆)Alkenyl substituiert ist, vorzugsweise 1*H*-pyrazol-4-yl ist, das an Position 1 mit Allyl substituiert ist.

13. Verbindung nach Anspruch 1, die aus der folgenden Liste ausgewählt ist:
*N*-Benzyl-1-(2-(6-methoxy-1,5-naphthyridin-4-yl)ethyl)piperidin-4-am in;
(4-((1-(2-(6-Methoxy-1,5-naphthyridin-4-yl)ethyl)piperidin-4-ylamino)methyl)phenyl)methanol;
4-((1-(2-(6-Methoxy-1,5-naphthyridin-4-yl)ethyl)piperidin-4-ylamino)methyl)benzamid;
*N*-(4-(Dimethylamino)benzyl)-1-(2-(6-methoxy-1,5-naphthyridin-4-yl)ethyl)piperidin-4-amin;
*N*-(4-Fluorbenzyl)-1-(2-(6-methoxy-1,5-naphthyridin-4-yl)ethyl)piperidin-4-amin;
*N*-(4-Chlorbenzyl)-1-(2-(6-methoxy-1,5-naphthyridin-4-yl)ethyl)piperidin-4-amin;
*N*-(4-Brom benzyl)-1-(2-(6-methoxy-1, 5-naphthyridin-4-yl)ethyl)piperid in-4-amin;
*N*-(4-Iodobenzyl)-1-(2-(6-methoxy-1,5-naphthyridin-4-yl)ethyl)piperidin-4-amin;
*N*-(4-(Difluormethoxy)-3-methoxybenzyl)-1-(2-(6-methoxy-1,5-naphthyridin-4-yl)ethyl)piperidin-4-amin;
2-(Difluormethoxy)-5-((1-(2-(6-methoxy-1,5-naphthyridin-4-yl)ethyl)piperidin-4-ylamino)methyl)phenol; und
*N*-((1-Allyl-1*H*-pyrazol-4-yl)methyl)-1-(2-(6-methoxy-1,5-naphthyridin-4-yl)ethyl)piperidin-4-amin.

14. Pharmazeutische Zusammensetzung umfassend eine Verbindung wie in einem der Ansprüche 1 bis 13 definiert oder ein pharmazeutisch verträgliches Salz davon, und einen pharmazeutisch verträglichen Träger, Hilfsstoff oder Vehikel.

15. Verbindung wie in einem der Ansprüche 1 bis 13 definiert, zur Verwendung als Arzneimittel, vorzugsweise zur Verwendung als Arzneimittel zur Behandlung einer bakteriellen Infektion.

## Revendications

1. Composé de la formule (I): 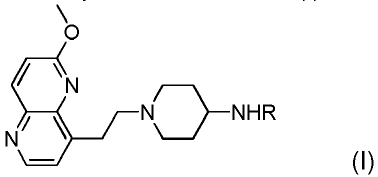 dans lequel R est un groupe -CH₂-R₁, R₁ étant un aryle monocyclique substitué ou non substitué ou un hétérocycle aromatique monocyclique substitué ou non substitué ;
ou un sel, un solvate, un hydrate ou un oxyde correspondant.

2. Composé selon la revendication 1, dans lequel R₁ est un aryle monocyclique non substitué, de préférence un phényle non substitué.

3. Composé selon la revendication 1, dans lequel R₁ est un aryle monocyclique, de préférence phényle, substitué par un ou plusieurs substituants choisis parmi OH, halogène, CR₃R₄R₅, NR₃R₄, OCR₃R₄R₅ et COR₆ ; dans lequel R₃, R₄ et R₅ sont indépendamment sélectionnés parmi H, OH, halogène et alkyle en (C₁₋₆), et R₆ est sélectionné parmi H, OH, halogène, alkyle en (C₁₋₆) et NR₇R₈, R₇ et R₈ étant indépendamment sélectionnés parmi H, halogène et alkyle en (C₁₋₆).

4. Composé selon la revendication 1, dans lequel R₁ est un aryle monocyclique, de préférence phényle, substitué par un ou plusieurs substituants choisis parmi OH, halogène, CH₂OH, C(O)NH₂, NH₂, N(CH₃)₂, OCH₃ et OCHR₉R₁₀, R₉ et R₁₀ étant chacun halogène.

5. Composé selon la revendication 4, dans lequel R₉ et R₁₀ sont chacun fluoro.

6. Composé selon l'une quelconque des revendications 3 à 5, dans lequel l'aryle monocyclique est monosubstitué, de préférence 4-monosubstitué, ou dans lequel l'aryle monocyclique est disubstitué, de préférence 3,4-disubstitué.

7. Composé selon la revendication 1, dans lequel R₁ est un hétérocycle aromatique monocyclique non substitué, de préférence un hétérocycle aromatique monocyclique non substitué à 5 ou 6 chaînons.

8. Composé selon la revendication 1, dans lequel R₁ est un hétérocycle aromatique monocyclique substitué, de préférence un hétérocycle aromatique substitué à 5 ou 6 chaînons, substitué par un ou plusieurs substituants choisis parmi alcényle en (C₂₋₆), diényle en (C₄₋₆), alcynyle en (C₂₋₆)et diynyle en (C₄₋₆).

9. Composé selon la revendication 8, dans lequel l'hétérocycle comporte un, deux ou trois hétéroatomes N, et dans lequel au moins un hétéroatome N est substitué par un ou plusieurs substituants choisis parmi alcényle en (C2-6), diényle (C4-6), alcynyle en (C2-6) et diynyle en (C4-6), ou dans lequel l'hétérocycle comporte un, deux ou trois hétéroatomes N, et dans lequel au moins un hétéroatome N est substitué par un ou plusieurs substituants choisis parmi éthényle, propényle, butényle, pentényle et hexényle, de préférence parmi vinyle, 2-propényle, 3-butényle, 4-pentényle et 5-hexényle.

10. Composé selon la revendication 8 ou 9, dans lequel l'hétérocycle aromatique monocyclique est monosubstitué.

11. Composé selon l'une quelconque des revendications 7 à 10, dans lequel l'hétérocycle aromatique monocyclique est un azole, de préférence l'hétérocycle aromatique monocyclique est un pyrazole, imidazole, 1,2,3-triazole ou 1,2,4-triazole, plus préférablement l'hétérocycle aromatique monocyclique est un pyrazole.

12. Composé selon l'une quelconque des revendications 7 à 11, dans lequel l'hétérocycle aromatique monocyclique est 1*H*-pyrazol-4-yl substitué en position 1 par alcényle en (C₂₋₆), de préférence 1*H*-pyrazol-4-yl substitué en position 1 par allyle.

13. Composé selon la revendication 1, qui est choisi dans la liste suivante :
*N*-benzyl-1-(2-(6-méthoxy-1,5-naphthyridin-4-yl)éthyl)pipéridin-4-amine ;
(4-((1-(2-(6-méthoxy-1,5-naphthyridin-4-yl)éthyl)pipéridin-4-ylamino)méthyl)phényl)méthanol ;
4-((1-(2-(6-méthoxy-1,5-naphthyridin-4-yl)éthyl)pipéridin-4-ylamino)méthyl)benzamide ;
*N*-(4-(diméthylamino)benzyl)-1-(2-(6-méthoxy-1,5-naphthyridin-4-yl)éthyl)pipéridin-4-amine ;
*N*-(4-fluorobenzyl)-1-(2-(6-méthoxy-1,5-naphthyridin-4-yl)éthyl)pipéridin-4-amine ;
*N*-(4-chlorobenzyl)-1-(2-(6-méthoxy-1,5-naphthyridin-4-yl)éthyl)pipéridin-4-amine ;
*N*-(4-bromobenzyl)-1-(2-(6-méthoxy-1,5-naphthyridin-4-yl)éthyl)pipéridin-4-amine ;
*N*-(4-iodobenzyl)-1-(2-(6-méthoxy-1,5-naphthyridin-4-yl)éthyl)pipéridin-4-amine ;
*N*-(4-(difluorométhoxy)-3-méthoxybenzyl)-1-(2-(6-méthoxy-1,5-naphthyridin-4-yl)éthyl)pipéridin-4-amine ;
2-(difluorométhoxy)-5-((1-(2-(6-méthoxy-1,5-naphthyridin-4-yl)éthyl)pipéridin-4-ylamino)méthyl)phénol ; et
*N*-((1-allyl-1*H*-pyrazol-4-yl)méthyl)-1-(2-(6-méthoxy-1,5-naphthyridin-4-yl)éthyl)pipéridin-4-amine.

14. Composition pharmaceutique, comprenant un composé tel que défini dans l'une quelconque des revendications 1 à 13 ou un sel pharmaceutiquement acceptable de celui-ci, et un support, adjuvant ou véhicule pharmaceutiquement acceptable.

15. Composé tel que défini dans l'une quelconque des revendications 1 à 13 pour une utilisation en tant que médicament, de préférence pour une utilisation en tant que médicament pour le traitement d'une infection bactérienne.
